# EUROPÄISCHE PATENTSCHRIFT

(11) **EP 0 384 895 B1**
(45) Veröffentlichungstag und Bekanntmachung des Hinweises auf die Patenterteilung: **06.12.1995**
(21) Anmeldenummer: 90810110.8
(22) Anmeldetag: 15.02.1990
(51) Int. Cl.: C07D 493/10, B41M 5/145, B41M 5/30

(54) **2- oder 3-Dicarbonsäureimid-Fluoranverbindungen, Verfahren zu deren Herstellung und deren Verwendung in Aufzeichnungsmaterialien**
2- Or 3-fluorane-dicarboxylic imides, process for their preparation and their use in recording materials
2-Ou 3-dicarboxamides fluoraniques, leur procédé de préparation et leur utilisation comme matériaux d'enregistrement

(30) Priorität: 24.02.1989 CH 675/89
(43) Veröffentlichungstag der Anmeldung: 29.08.1990
(73) Patentinhaber: CIBA-GEIGY AG, 4002 Basel (CH)
(72) Erfinder: Zink, Rudolf, CH-4106 Therwil (CH)

(56) Entgegenhaltungen:
- DE-A- 2 542 365
- CHEMICAL ABSTRACTS, Band 95, Nr. 18, 2. November 1981, Seiten 619-620,Zusammenfassung Nr. 159847q, Columbus, Ohio, US;& JP-A-56 030 893 (SUMITOMO CHEMICAL CO., LTD) 28-03-1981

## Beschreibung

Die vorliegende Erfindung betrifft 2- oder 3-Dicarbonsäureimid-Fluoranverbindungen, Verfahren zu ihrer Herstellung und ihre Verwendung als Farbbildner in druckempfindlichen oder wärmeempfindlichen Aufzeichungsmaterialien.

In Chem. Abs. 95, Abs. No. 159847q (1981) werden Fluorarverbindungen offenbart, die in 2-Stellung der Fluorangruppe durch eine über eine - NH-Ph-CH₂-Brücke gebundene Dicarbonsäureimidgruppe substituiert sind. Die Verbindungen eignen sich als Farbbildner in druck- oder wärmeempfindlichen Aufzeichnungsmaterialien.

Die DE-A-2,542,365 offenbart Fluorane, die in 2-Stellung durch eine N'-Phenyl-N-phenylureido-Gruppe substituiert sind. Die Fluorane eignen sich für druckempfindliche Kopiersysteme.

Die erfindungsgemässen Fluoranverbindungen entsprechen der allgemeinen Formel 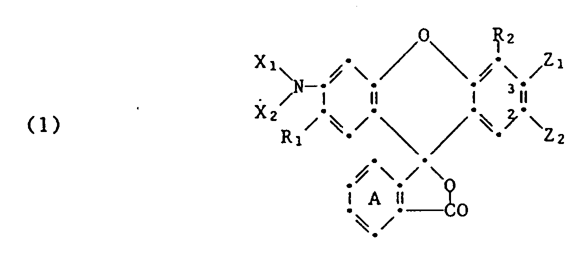
worin
- R₁ und R₂,: unabhängig voneinander, je Wasserstoff, Halogen, Niederalkyl oder Niederalkoxy,
von Z₁ und Z₂ eines die Dicarbonsäureimidgruppe 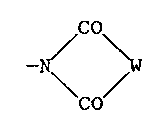
und das andere Wasserstoff, Halogen, C₁-C₆-Alkyl oder C₁-C₆-Alkoxy,
- W: einen gegebenenfalls durch Halogen substituierten, geradkettigen oder verzweigten Kohlenwasserstoffrest einer gesättigten oder ethylenisch ungesättigten Dicarbonsäure mit 4 bis 10 Kohlenstoffatomen, einen zweiwertigen cycloaliphatischen Rest oder den zweiwertigen Rest einer aromatischen Dicarbonsäure,
- X₁ und X₂,: unabhängig voneinander, je Wasserstoff, unsubstituiertes oder, durch Halogen, Hydroxy, Cyano, Tetrahydrofuryl oder C₁-C₆-Alkoxy substituiertes Alkyl mit höchstens 12 Kohlenstoffatomen, Cycloalkyl mit 5 bis 10 Kohlenstoffatomen oder unsubstituiertes oder durch Halogen, Cyano, Nitro, C₁-C₆-Alkyl, C₁-C₆-Alkoxy, C₁-C₆-Alkoxycarbonyl, -NX'X'' oder 4-NX'X''-phenylamino substituiertes Benzyl oder Phenyl, worin X' und X'', unabhängig voneinander, Wasserstoff, C₁-C₆-Alkyl, Cyclohexyl, Benzyl oder Phenyl darstellen, oder
- X₁ und X₂: zusammen mit dem sie verbindenden Stickstoffatom einen fünf- oder sechsgliedrigen, vorzugsweise gesättigten, heterocyclischen Rest bedeuten und worin der Ring A unsubstituiert oder durch Halogen, Nitro, C₁-C₆-Alkyl, C₁-C₆-Alkoxy, C₁-C₆-Alkylthio, C₁-C₆-Alkoxycarbonyl, Amino, MonoC₁-C₆-Alkylamino oder Di-C₁-C₆-Alkylamino substituiert ist.
Die 2-Dicarbonsäureimidfluorane sind bevorzugt.

Beispiele für C₁-C₆-Alkyl-, -Alkoxy- und Alkylthio-Gruppen sind Methyl, Ethyl, n-Propyl, Isopropyl, n-Butyl, sek.-Butyl, tert.Butyl, Amyl, Isoamyl, tert.Amyl, Hexyl, Methoxy, Ethoxy, Isopropoxy, Isobutoxy oder tert.Butoxy bzw. Methylthio, Ethylthio, Propylthio oder Butylthio.

Halogen bedeutet beispielsweise Fluor, Brom oder vorzugsweise Chlor.

In der Bedeutung eines zweiwertigen, aliphatischen Restes stellt W vorteilhafterweise einen gegebenenfalls durch Halogen substituierten, geradkettigen oder verzweigten Kohlenwasserstoffrest einer gesättigten oder ethylenisch-ungesättigten Dicarbonsäure mit 4 bis 10 Kohlenstoffatomen dar. Als derartige aliphatische, gesättigte Dicarbonsäuren kommen z.B. Bernstein- oder Glutarsäure in Betracht. Ethylenisch-ungesättigte Dicarbonsäuren sind vorzugsweise die Malein-, Dimethylmalein-, Dichlormalein-, Itacon-, Citracon- oder Glutaconsäure.

In der Bedeutung eines zweiwertigen cycloaliphatischen Restes stellt W insbesondere den Rest einer Tetrahydro- oder Hexahydrophthalsäure dar.

Vorzugsweise stellt W den zweiwertigen Rest einer aromatischen Dicarbonsäure, wie z.B. einer Naphthalindicarbonsäure oder einer o-Phthalsäure dar.

Bevorzugt stellt W einen Phenylenrest dar, welcher mit Nitro, Halogen, wie z.B. Chlor oder Brom, C₁-C₆-Alkyl, C₁-C₆-Alkoxy oder C₁-C₆-Alkoxycarbonyl substituiert sein kann. Insbesondere bedeutet W einen unsubstituierten Phenylenrest.

R₁ und R₂ sowie ein Z sind vorzugsweise Wasserstoff, Methyl, Methoxy oder Chlor.

Stellen die Substituenten X₁ und X₂ Alkylgruppen dar, so können sie geradkettig oder verzweigt sein. Beispiele solcher Alkylreste sind Methyl, Ethyl, n-Propyl, Isopropyl, n-Butyl, sek.-Butyl, tert.-Butyl, 1,1,3,3-Tetramethylbutyl, Amyl, Isoamyl, n-Hexyl, 2-Ethyl-hexyl, n-Heptyl, n-Octyl, Isooctyl, n-Nonyl, Isononyl oder n-Dodecyl.

Sind die Alkylreste in X₁ und X₂ substituiert, so handelt es sich vor allem um Cyanoalkyl, Halogenalkyl, Hydroxyalkyl, Alkoxyalkyl jeweils vorzugsweise mit insgesamt 2 bis 8 Kohlenstoffatomen, wie z.B. β-Cyanoethyl, β-Chlorethyl, γ-Chlorpropyl, β-Hydroxyethyl, γ-Hydroxypropyl, β-Methoxyethyl, β-Ethoxyethyl oder γ-Methoxypropyl. Ein weiterer substituierter Alkylrest ist Tetrahydrofurfuryl.

Beispiele für Cycloalkyl in der Bedeutung der X-Reste sind Cyclopentyl, Cycloheptyl oder vorzugsweise Cyclohexyl. Die Cycloalkylreste können einen oder mehrere C₁-C₄-Alkylreste, vorzugsweise Methylgruppen, enthalten und weisen insgesamt 5 bis 10 Kohlenstoffatome auf.

Bevorzugte Substituenten in der Benzyl- und Phenylgruppe der X-Reste sind z.B. Halogen, Cyano, Methyl, Methoxy oder Carbomethoxy. Beispiele für derartige araliphatische bzw. aromatische Reste sind Methylbenzyl, 2,4- oder 2,5-Dimethylbenzyl, Chlorbenzyl, Dichlorbenzyl, Cyanobenzyl, Tolyl, Xylyl, 2,6-Dimethylphenyl, Chlorphenyl, Nethoxyphenyl oder Carbomethoxyphenyl.

Wenn die Substituenten (X₁ und X₂) zusammen mit dem gemeinsamen Stickstoffatom einen heterocyclischen Rest darstellen, so ist dieser beispielsweise Pyrrolidino, Piperidino, Pipecolino, Morpholino, Thiomorpholino oder Piperazino, z.B. N-Methylpiperazino. Bevorzugte gesättigte heterocyclische Reste für -NX₁X₂ sind Pyrrolidino, Piperidino oder Morpholino.

Die Substituenten X₁ und X₂ sind vorzugsweise Cyclohexyl, Tolyl, Xylyl, Benzyl, Cyano-C₁-C₆-Alkyl z.B. β-Cyanoethyl oder in erster Linie C₁-C₆-Alkyl, wie z.B. Methyl, Ethyl, n-Propyl, Isopropyl, n-Butyl, Amyl, Hexyl. -NX₁X₂ ist bevorzugt auch Pyrrolidinyl, N-C₁-C₆-Alkyl-N-tetrahydrofurfurylamino, 4-DiC₁-C₆-Alkylaminophenylamino oder 4-(4'-Phenylaminophenylamino)-phenylamino.

Der Ring A kann als Substituent vorteilhafterweise Halogen, Nitro, C₁-C₆-Alkyl, C₁-C₆-Alkoxy oder DiC₁-C₆-Alkylamino enthalten. Vorzugsweise ist der Benzolring A unsubstituiert oder durch 1 bis 4 Halogenatome substituiert.

Praktisch wichtige Dicarbonsäureimid-Fluoranverbindungen entsprechen der Formel 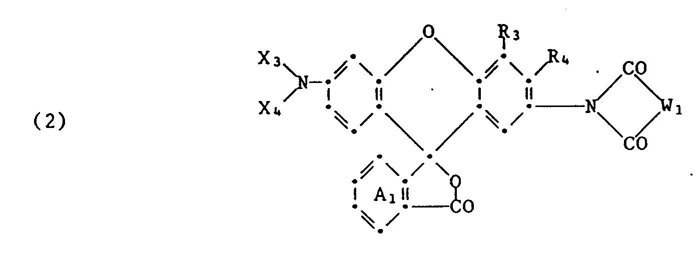
oder
der Formel 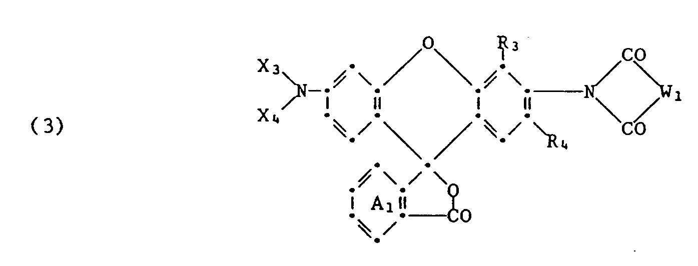
worin
- W₁: Alkylen oder Alkenylen mit 2 bis 4 Kohlenstoffatomen, Cyclohexylen oder Phenylen, das unsubstituiert oder durch Halogen, wie z.B. Chlor oder Brom, Methyl, Methoxy oder Carbomethoxy substituiert ist,
- R₃ und R₄,: unabhängig voneinander, je Wasserstoff, Halogen oder C₁-C₆-Alkyl,
- X₃: C₁-C₆-Alkyl, C₅-C₆-Cycloalkyl, Benzyl, Phenyl oder durch Halogen, C₁-C₄-Alkyl oder C₁-C₄-Alkoxy substituiertes Phenyl, X₄ C₁-C₆-Alkyl oder Benzyl oder -NX₃X₄ Pyrrolidinyl, Piperidinyl, Morpholinyl oder auch N-C₁-C₆-Alkyl-N-tetrahydrofurfurylamino
bedeuten und worin
der Benzolring A₁ unsubstituiert oder durch Halogen, C₁-C₆-Alkyl oder C₁-C₆-Alkoxy substituiert ist.

Unter den Verbindungen der Formeln (2) und (3) sind die Fluoranverbindungen, in denen W₁ unsubstituiertes oder durch Chlor substituiertes Phenylen, X₃ und X₄ C₁-C₄-Alkyl,
R₃ und R₄ Wasserstoff, Methyl oder Chlor bedeuten und der Ring A₁ unsubstituiert ist, bevorzugt.

Von besonderem Interesse sind Fluoranverbindungen der Formel 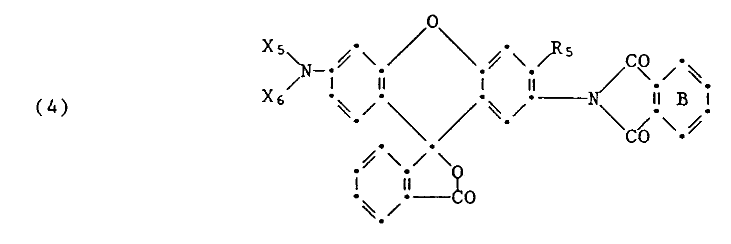
worin
- R₅: Wasserstoff oder Methyl,
- X₅: C₁-C₄-Alkyl, Cyclohexyl oder Tolyl
und
- X₆: C₁-C₄-Alkyl bedeuten und der Ring B unsubstituiert oder durch 1 bis 4 Chloratome substituiert ist.

Die erfindungsgemässen Fluoranverbindungen der Formeln (1) bis (4) werden dadurch hergestellt, dass man eine Ketosäureverbindung der Formel 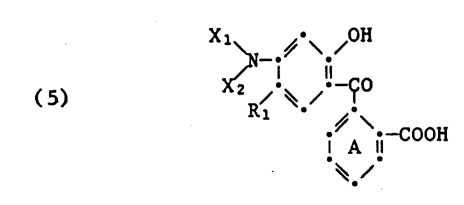
worin A, X₁, X₂ und R₁ die angegebene Bedeutung haben, mit einer die Imidgruppe enthaltenden Verbindung der Formel 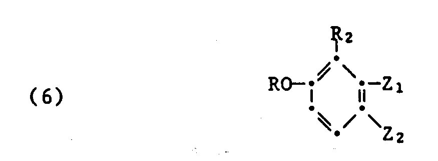
worin R₂, Z₁ und Z₂ die angegebene Bedeutung haben und R Wasserstoff oder Methyl bedeutet, umsetzt.

Die Umsetzung wird vorzugsweise so ausgeführt, dass man die Reaktionskomponenten in Anwesenheit eines sauren Kondensationsmittels bei einer Temperatur von 20 bis 140°C zur Reaktion bringt. Beispiele für derartige Kondensationsmittel sind Essigsäureanhydrid, Zinkchlorid, Aluminiumchlorid, Schwefelsäure, Phosphorsäure und Phosphoroxychlorid.

Die Isolierung des Endproduktes der Formel (1) erfolgt in allgemein bekannter Weise durch Einstellen des Reaktionsgemisches auf einen pH-Wert von mindestens 6, vorzugsweise 7 bis 14, z.B. mit Alkalien, wie z.B. Alkalimetallhydroxiden, Ammoniak, Alkalimetallcarbonaten oder -bicarbonaten und Abtrennen des gebildeten Produktes, Waschen und Trocknen oder durch Behandeln mit geeigneten organischen Lösungsmitteln, wie z.B. Methanol, Isopropanol, Benzol, Chlorbenzol, Toluol oder Xylol. Nötigenfalls kann zum Umkristallisieren der Fluoranverbindungen auch Petrolether, Wasser oder Ammoniakwasser mitverwendet werden.

Die Ausgangsprodukte der Formeln (5) und (6) sind grösstenteils bekannt.

In einem Alternativverfahren können die erfindungsgemässen Fluoranverbindungen der Formeln (1) bis (4) dadurch hergestellt werden, dass man eine Amino-Fluoranverbindung der Formel 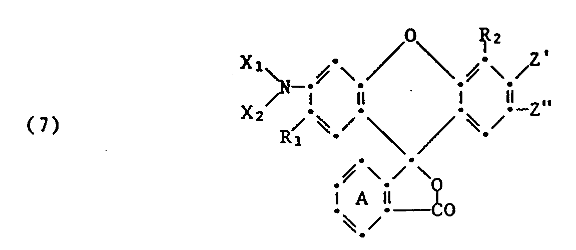
worin von Z' und Z'' eines -NH₂ und das andere Wasserstoff, Halogen, C₁-C₆-Alkyl oder C₁-C₆-Alkoxy bedeuten und R₁, R₂, X₁, X₂ und A die angegebene Bedeutung haben, mit einem Dicarbonsäureanhydrid der Formel 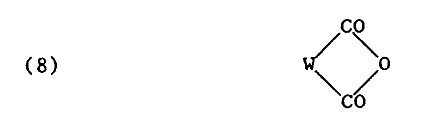
worin W die angegebene Bedeutung hat, umsetzt.

Spezifische Beispiele für Anhydridkomponenten der Formel (8) sind Bernsteinsäureanhydrid, Glutarsäureanhydrid, Maleinsäureanhydrid, Dimethylmaleinsäureanhydrid, Dichlormaleinsäureanhydrid, Citraconsaureanhydrid, Itaconsäureanhydrid, Phthalsäureanhydrid, Tetrachlorphthalsäureanhydrid, 1,8-Naphthalsäureanhydrid oder 1,4,5,6,7,7-Hexachlor-5-norbornen-2,3-dicarbonsäureanhydrid.

Die Umsetzung der Fluoranverbindung der Formel (7) mit der Dicarbonsäureanhydridverbindung der Formel (8) kann bei einer Temperatur von 10 bis 140°C vorgenommen werden. Als Reaktionsmedium kann ein organisches Lösungsmittel und/oder eine niedere aliphatische Carbonsäure, wie z.B. Essigsäure, verwendet werden.

Als Lösungsmittel kommen beispielsweise cycloaliphatische oder aromatische Kohlenwasserstoffe, wie z.B. Cyclohexan, Benzol, Toluol oder Xylol; Chlorkohlenwasserstoffe, wie Chloroform, Ethylenchlorid oder Chlorbenzol; Ether, wie Diethylether oder Glykoldimethylether; cyclische Ether, wie Dioxan oder Tetrahydrofuran; sowie Dimethylformamid, Diethylformamid, Dimethylsulfoxid oder Acetonitril in Betracht.

Die Fluoranverbindungen der Formeln (1) bis (4) sind normalerweise farblos oder höchstens schwach gefärbt. Wenn diese Farbbildner mit einem vorzugsweise sauren Entwickler, d.h. einem Elektronenakzeptor, in Kontakt gebracht werden, so ergeben sie meistens intensiv gelbe, orange oder rote Farbtöne, die besonders lichtecht sind.

Die Fluoranverbindungen der Formeln (1) bis (4) sind auch sehr wertvoll im Gemisch mit einem oder mehreren anderen bekannten Farbbildnern, z.B. 3,3-(Bis-aminophenyl-)-phthaliden, 3-Indolyl-3-aminophenylazaphthaliden, (3,3-Bis-indolyl-)-phthaliden, 3-Aminofluoranen, 6-Dialkylamino-2-dibenzylaminofluoranen, 6-Dialkylamino-3-methyl-2-arylaminofluoranen, 3,6-Bisalkoxyfluoranen, Leukoauraminen, Spiropyranen, Spirodipyranen, Chromenopyrazolen, Chromenoindolen, Phenoxazinen, Phenothiazinen, Chinazolinen, Rhodaminlaktamen, Carbazolylmethanen oder weiteren Triarylmethan-leukofarbstoffen, um graue oder schwarze Färbungen zu erhalten.

Die Fluoranverbindungen der Formeln (1) bis (4) zeigen sowohl auf aktivierten Tonen, wie auch auf phenolischen Unterlagen eine ausgezeichnete Farbintensität. Sie eignen sich insbesondere als schnell entwickelnde Farbbildner für die Verwendung in einem wärmeempfindlichen oder insbesondere druckempfindlichen Aufzeichnungsmaterial, das sowohl Kopier-als auch Registriermaterial sein kann. Sie zeichnen sich dadurch aus, dass sie pH stabil und in den Kapselölen gut löslich sind. Nach Belichtung im CB-Blatt weisen sie eine geringe Abnahme der Farbstärke (CB-Desaktivierung) auf. Die Sublimationsechtheit ist hervorragend.

Ein druckempfindliches Material besteht beispielsweise aus mindestens einem Paar von Blättern, die mindestens einen Farbbildner der Formeln (1) bis (4) gelöst in einem organischen Lösungsmittel und einen Elektronenakzeptor als Entwickler enthalten.

Typische Beispiele für solche Entwickler sind Aktivton-Substanzen, wie z.B. Attapulgus-Ton, Säureton, Bentonit, Montmorillonit, aktivierter Ton, wie z.B. säureaktiviertes Bentonit oder Montmorillonit, ferner Zeolith, Halloysit, Siliciumdioxyd, Aluminiumoxid, Aluminiumsulfat, Aluminiumphosphat, Zinkchlorid, Zinknitrat, Zirkondioxid, aktiviertes Kaolin oder irgendein beliebiger Ton. Als Entwickler können auch sauer reagierende, organische Verbindungen, wie z.B. gegebenenfalls ringsbustituierte Phenole, Resorcine, Salicylsäuren, wie z.B. 3,5-Bis-(α,α-dimethylbenzyl-)-salicylsäure oder 3,5-Bis-(α-methylbenzyl)-salicylsäure oder Salicylsäureester und deren Metallsalze, z.B. Zinksalze, sowie ein sauer reagierendes, polymeres Material, wie z.B. ein phenolisches Polymerisat, ein Alkylphenolacetylenharz, ein Maleinsäure-Kolophonium-Harz oder ein teilweise oder vollständig hydrolysiertes Polymerisat von Maleinsäureanhydrid mit Styrol, Ethylen oder Vinylmethylether, oder Carboxymethylen verwendet werden. Es können auch Mischungen der genannten monomeren und polymeren Verbindungen eingesetzt werden. Besonders bevorzugte Entwickler sind säureaktiviertes Bentonit, Zinksalicylate oder die Kondensationsprodukte von p-substituierten Phenolen mit Formaldehyd. Die letzteren können auch mit Zink modifiziert sein.

Die Entwickler können zusätzlich auch im Gemisch mit an sich unreaktiven oder wenig reaktiven Pigmenten oder weiteren Hilfstoffen wie Kieselgel oder UV-Absorbern, wie z.B. 2-(2′-Hydroxyphenyl-)benztriazolen eingesetzt werden. Beispiele für solche Pigmente sind: Talk, Titandioxid, Aluminiumoxid, Aluminiumhydroxyd, Zinkoxid, Kreide, Tone wie Kaolin, sowie organische Pigmente, z.B. Harnstoff-Formaldehydkondensate (BET-Oberfläche 2-75 m²/g) oder Melamin-Formaldehyd-Kondensationsprodukte.

Der Farbbildner liefert an den Punkten, an denen er mit dem Elektronenakzeptor in Kontakt kommt, eine gefärbte Markierung. Um eine frühzeitige Aktivierung der im druckempfindlichen Aufzeichnungsmaterial vorhandenen Farbbildner zu verhindern, werden diese in der Regel vom Elektronenakzeptor getrennt. Dies kann zweckmässig erzielt werden, indem man die Farbbildner in schaum-, schwamm- oder bienenwabenartigen Strukturen einarbeitet. Vorzugsweise sind die Farbbildner in Mikrokapseln eingeschlossen, die sich in der Regel durch Druck zerbrechen lassen.

Beim Zerbrechen der Kapseln durch Druck, beispielsweise mittels eines Bleistiftes, wird die Farbbildnerlösung auf ein benachbartes mit einem Elektronenakzeptor beschichtetes Blatt übertragen, wodurch eine farbige Stelle erzeugt wird. Die Farbe resultiert aus dem dabei gebildeten Farbstoff, der im sichtbaren Bereich des elektromagnetischen Spektrums absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z.B. halogeniertes Benzol, Diphenyl oder Paraffin, wie Chlorparaffin, Trichlorbenzol, Monochlordiphenyl, Dichlordiphenyl oder Trichlordiphenyl; Ester, wie z.B. Tricresylphosphat, Di-n-butylphthalat, Dioctylphthalat, Trichlorethylphosphat; aromatische Ether, wie Benzylphenylether, Kohlenwasserstofföle, wie Paraffin oder Kerosin, z.B. mit Isopropyl, Isobutyl, sek-Butyl oder tert.-Butyl alkylierte Derivate von Diphenyl, Naphthalin oder Terphenyl, Dibenzyltoluol, partiell hydriertes Terphenyl, mono- bis tetra-C₁-C₃-alkylierte Diphenylalkane, Dodecylbenzol, benzylierte Xylole, oder weitere chlorierte oder hydrierte, kondensierte, aromatische Kohlenwasserstoffe. 0ft werden Mischungen verschiedener Lösungsmittel, insbesondere Mischungen aus Paraffinölen oder Kerosin und Diisopropylnaphthalin oder partiell hydriertem Terphenyl, eingesetzt, um eine optimale Löslichkeit für die Farbbildung, eine rasche und intensive Färbung und eine für die Mikroverkapselung günstige Viskosität zu erreichen. Bei der Einkapselung zeichnen sich die erfindungsgemässen Fluoranverbindungen dadurch aus, dass sie ausserordentlich hohe pH-Beständigkeit z.B. in einem pH-Bereich von 4 bis 10 zeigen.

Die Kapselwände können durch Koazervationskräfte gleichmässig um die Tröpfchen der Farbbildnerlösung herum gebildet werden, wobei das Einkapselungsmaterial z.B. in der US-Patentschrift 2,800,457 beschrieben ist. Die Kapseln können vorzugsweise auch aus einem Aminoplast oder modifizierten Aminoplasten durch Polykondensation gebildet werden, wie es in den britischen Patentschriften 989,264, 1,156,725, 1,301,052 und 1,355,124 beschrieben ist. Ebenfalls geeignet sind Mikrokapseln, welche durch Grenzflächenpolymerisation gebildet werden, wie z.B. Kapseln aus Polyester, Polycarbonat, Polysulfonamid, Polysulfonat, besonders aber aus Polyamid oder Polyurethan.

Die Farbbildner der Formeln (1) bis (4) enthaltenden Mikrokapseln können zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten und durch das Trägermaterial.

Bevorzugt wird eine Anordnung, bei welcher der eingekapselte Farbbildner in Form einer Schicht auf der Rückseite eines Uebertragungsblattes und der Elektronenakzeptor in Form einer Schicht auf der Vorderseite eines Empfangsblattes vorhanden sind. Eine andere Anordnung der Bestandteile besteht darin, dass die den Farbbildner enthaltenden Mikrokapseln und der Entwickler in oder auf dem gleichen Blatt in Form einer oder mehrerer Einzelschichten oder in der Papierpulpe vorliegen.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel, wie Gummi arabicum, Polyvinylalkohol, Hydroxymethylcellulose, Casein, Methylcellulose, Dextrin, Stärke, Stärkederivate oder Polymerlatices. Letztere sind beispielsweise Butadien-Styrolcopolymerisate oder Acrylhomo- oder -copolymere.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet. Schichtträger kann auch eine Kunstfolie sein.

Vorzugsweise besteht das Durchschreibematerial auch darin, dass es eine kapselfreie, den Farbbildner enthaltende Schicht und eine farbentwickelnde Schicht, die als Farbentwickler mindestens ein anorganisches Metallsalz eines mehrwertigen Metalles, vor allem Halogenide oder Nitrate, wie z.B. Zinkchlorid, Zinnchlorid, Zinknitrat oder deren Gemische enthält, aufweist.

Die Verbindungen der Formeln (1) bis (4) können auch als Farbbildner in einem thermoreaktiven Aufzeichnungsmaterial verwendet werden. Dieses enthält in der Regel mindestens einen Schichtträger, einen oder mehrere Farbbildner, einen Elektronenakzeptor und gegebenenfalls auch ein Bindemittel und/oder Wachs. Gewünschtenfalls können auch Aktivatoren oder Sensibilisatoren im Aufzeichnungsmaterial vorhanden sein.

Thermoreaktive Aufzeichnungssysteme umfassen, z.B. wärmeempfindliche Aufzeichnungs- und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Messinstrumenten, wie z.B. Elektrocardiographen, verwendet. Die Bilderzeugung (Markierung) kann auch manuell mit einer erhitzten Feder erfolgen. Eine weitere Einrichtung zur Erzeugung von Markierungen mittels Wärme sind Laserstrahlen.

Das thermoreaktive Aufzeichnungsmaterial kann so aufgebaut sein, dass der Farbbildner in einer Bindemittelschicht gelöst oder dispergiert ist und in einer zweiten Schicht der Entwickler im Bindemittel gelöst und dispergiert ist. Eine andere Möglichkeit besteht darin, dass sowohl der Farbbildner als auch der Entwickler in einer Schicht dispergiert sind. Die Schicht bzw. Schichten werden in spezifischen Bezirken mittels Wärme erweicht, worauf sich sofort in den erwärmten Teilen die erwünschte Farbe entwickelt.

Als Entwickler eignen sich die gleichen Elektronenakzeptoren, wie sie in druckempfindlichen Papieren verwendet werden. Beispiele für Entwickler sind die bereits erwähnten Tonminerale und Phenolharze, oder auch phenolische Verbindungen, wie sie beispielsweise in der DE-PS 12,51,348 beschrieben sind, z.B. 4-tert.-Butylphenol, 4-Phenylphenol, Methylen-bis-(p-phenylphenol), 4-Hydroxydiphenylether, α-Naphthol, β-Naphthol, 4-Hydroxybenzoesäure-methylester oder -benzylester, 4-Hydroxydiphenylsulfon, 2,4-Dihydroxydiphenylsulfon, 4′-Hydroxy-4-methyldiphenylsulfon, 4′-Hydroxy-4-isopropoxydiphenylsulfon, 4-Hydroxy-acetophenon, 2,2′-Dihydroxydiphenyl, 4,4′-Cyclohexylidendiphenol, 4,4′-Isopropylidendiphenol, 4,4′-Isopropyliden-bis-(2-methylphenol), ein Antipyrinkomplex von Zinkthiocyanat, ein Pyridinkomplex von Zinkthiocyanat, ein Kresidinkomplex von Zinkthiocyanat, 4,4-Bis-(4-hydroxyphenyl)valeriansäure, Hydrochinon, Pyrogallol, Phloroglucin, p-, m-, o-Hydroxybenzoesäure, Gallussäure, 1-Hydroxy-2-naphthoesäure, Hydroxyphthalsäure, sowie Borsäure oder organische, vorzugsweise aliphatische Dicarbonsäuren, wie z.B. Weinsäure, Oxalsäure, Maleinsäure, Zitronensäure, Citraconsäure oder Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Fluoranverbindungen und der Entwickler in Wasser schwer löslich oder unlöslich sind. Das Bindemittel sollte in der Lage sein, den Farbbildner und den Entwickler bei Raumtemperatur zu dispergieren und zu fixieren.

Wasserlösliche oder mindestens in Wasser quellbare Bindemittel sind z.B. hydrophile Polymerisate, wie Polyvinylalkohol, Polyacrylsäure, Hydroxyethylcellulose, Methylcellulose, Carboxymethylcellulose, Polyacrylamid, Polyvinylpyrrolidon, carboxylierte Butadien-Styrolcopolymerisate, Gelatine, Stärke oder veretherte Maisstärke.

Wenn der Farbbildner und der Entwickler in zwei getrennten Schichten vorliegen, können in Wasser unlösliche Bindemittel, d.h. in nichtpolaren oder nur schwach polaren Lösungsmitteln lösliche Bindemittel, wie z.B. Naturkautschuk, synthetischer Kautschuk, chlorierter Kautschuk, Polystyrol, Styrol/Butadien-Mischpolymerisate, Polymethylacrylate, Ethylcellulose, Nitrocellulose und Polyvinylcarbazol, verwendet werden. Die bevorzugte Anordnung ist jedoch diejenige, bei welcher der Farbbildner und der Entwickler in einer Schicht in einem wasserlöslichen Bindemittel enthalten sind.

Um die Stabilität des wärmeempfindlichen Aufzeichnungsmaterials oder die Bilddichte des entwickelten Bildes zu gewährleisten, kann das Material mit einer zusätzlichen Schutzschicht versehen sein. Derartige Schutzschichten bestehen in der Regel aus wasserlöslichen und/oder wasserunlöslichen Harzen, die herkömmliche Polymermaterialien oder wässrige Emulsionen von diesen Polymermaterialien sind.

Sowohl die thermoreaktiven Schichten als auch die Harzschichten können weitere Zusätze enthalten. Zur Verbesserung des Weissgrades, zur Erleichterung des Bedruckens der Papiere und zur Verhinderung des Festklebens der erhitzten Feder können diese Schichten, z.B. Talk, Titandioxyd, Zinkoxyd, Aluminiumhydroxyd, Calciumcarbonat (z.B. Kreide), Magnesiumcarbonat, Tone oder auch organische Pigmente, wie z.B. Harnstoff-Formaldehydpolymerisate enthalten. Um zu bewirken, dass nur innnerhalb eines begrenzten Temperaturbereiches die Farbe gebildet wird, können Substanzen, wie Harnstoff, Thioharnstoff, Diphenylthioharnstoff, Acetamid, Acetanilid, Benzolsulfanilid, Bis-Stearoyl-ethylendiamid, Stearinsäureamid, Phthalsäureanhydrid, Metallstearate, wie z.B. Zinkstearat, Phthalsäurenitril, Benzyldiphenyl, Dimethylterephthalat, Dibenzylterephthalat oder andere entsprechende, schmelzbare Produkte, welche das gleichzeitige Schmelzen des Farbbildners und des Entwicklers induzieren, zugesetzt werden. Bevorzugt enthalten thermographische Aufzeichnungsmaterialien Wachse, z.B. Carnaubawachs, Montanwachs, Paraffinwachs, Polyethylenwachs, Kondensate höherer Fettsäureamide und Formaldehyde oder Kondensate höherer Fettsäuren und Ethylendiamin.

Eine weitere Anwendung der Verbindungen der Formeln (1) bis (4) ist die Herstellung eines Farbbildes mittels photohärtbarer Mikrokapseln, wie sie z.B. in der DE-OS 3 247 488 beschrieben sind.

In den folgenden Beispielen beziehen sich die angegebenen Prozentsätze, wenn nichts anderes angegeben ist, auf das Gewicht. Teile bedeuten Gewichtsteile.

Beispiel 1: 7,8 g 2′-Carboxy-2-hydroxy-4-diethylamino-benzophenon werden bei 10°C in 55 g Schwefelsäure (100 %) gelöst. Man trägt bei 5-10°C innerhalb von 20 Minuten 6,3 g N-(4-Methoxy-phenyl)-phthalimid ein und lässt 1 Stunde bei 20-22°C rühren. Die entstandene Lösung wird auf 250 ml Eiswasser gegossen, die erhaltene Suspension nach 1 Stunde bei 22°C filtriert und mit kaltem Wasser gewaschen. 50 g des Filtriergutes werden in 100 ml Toluol mit 6,9 g Kaliumcarbonat (fein gemahlen) suspendiert. Man heizt auf Rückfluss (82°C) und kreist mittels Wasserabscheider das Wasser innerhalb von 2 Stunden aus, wobei die Rückflusstemperatur auf 105°C steigt. Die Toluolphase wird anschliessend mehrmals mit Wasser extrahiert und klärfiltriert. Durch Einengen erhält man 9 g der Fluoranverbindung der Formel 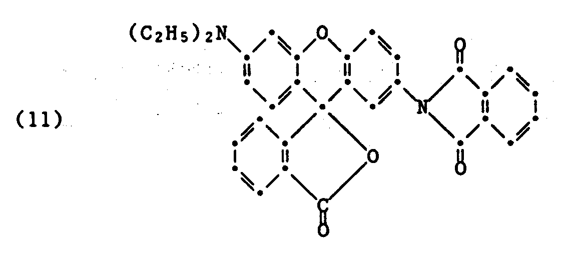
gelbliche Kristalle, Smp.: 221-222°C.

Dieser Farbbildner zeichnet sich durch eine hohe Sublimationsechtheit aus und entwickelt auf Aktivton sofort eine rote Farbe.

Beispiel 2: Vefährt man analog Beispiel 1, setzt aber anstelle von 6,3 g N-(4-Methoxy-phenyl)-phthalimid, 6,7 g N-(4-Methoxy-2-methylphenyl)-phthalimid ein, erhält man die Fluoranverbindung der Formel 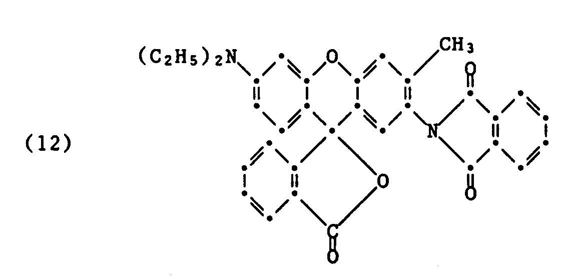
mit einem Schmelzpunkt von 260-262°C. Dieser sublimierechte Farbbildner entwickelt auf Aktivton sofort eine orange Farbe.

Beispiel 3: 7,7 g 2-Amino-6-diethylaminofluoran werden in 75 ml Eisessig bei 25°C gelöst. Innert 15 Minuten werden 5,7 g Tetrachlorphthalsäureanhydrid unter gutem Rühren eingetragen, wobei die Temperatur auf 32°C ansteigt. Man heizt auf 80°C und hält diese Temperatur während 10 Minuten. Der Ringschluss zum Imid wird mittels Dünnschichtchromatographie kontrolliert. Die Reaktionslösung wird unter Rühren langsam abgekühlt, wobei das Produkt ab 75°C auskristallisiert. Nach 3 Stunden filtriert man bei 25°C ab und wäscht das Filtriergut mit 1 l destilliertem Wasser. Nach Trocknung erhält man 12,2 g einer Fluoranverbindung der Formel 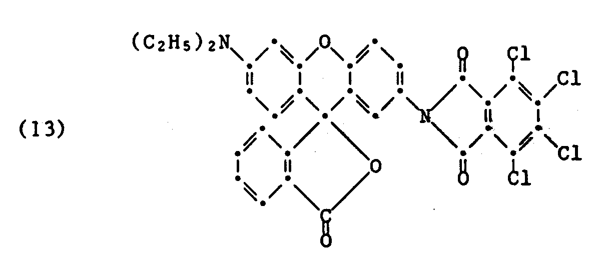
Umkristallisiert aus Xylol (Isomerengemisch) erhält man das reine Produkt mit einem Schmelzpunkt von 285-287°C. Auf Aktivton erzeugt man damit eine instante, rote Farbe.

Beispiel 4: 27,7 g 2′-Carboxy-2-hydroxy-4-n-dibutylamino-benzophenon werden in 150 g Schwefelsäure-Monohydrat bei 45°C gelöst. Bei 5-10°C trägt man innerhalb von 15 Minuten 19 g N-(4-Methoxy-phenyl)-phthalimid ein und rührt zuerst 1 Stunde bei 0-10°C, erwärmt auf 25°C und rührt dann eine weitere Stunde bei 25-30°C. Die entstandene Lösung wird auf 750 ml Eiswasser ausgetragen, wobei das Produkt ausfällt. Man rührt die entstandene Suspension noch während 15 Stunden bei 20-25°C, stellt den pH-Wert mit 40%iger Natriumhydroxidlösung auf 10, wobei auch die Temperatur auf 55°C gestellt wird, rührt noch 1 Stunde unter diesen Bedingungen und filtriert ab.

Das erhaltene, feuchte Produkt trägt man in ein Gemisch von 150 ml Xylol (Isomerengemisch) und 13,8 g Kaliumcarbonat ein, erwärmt zum Sieden und destilliert Wasser azeotrop ab, bis eine Siedetemperatur von 122°C erreicht wird. Zur Aufarbeitung gibt man 200 ml Wasser zu, trennt die Xylolphase und engt sie zur Trockene ein. Man erhält 26,4 g einer Verbindung der Formel 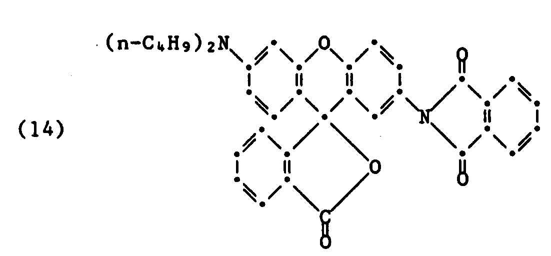
welche nach Umkristallisation aus 3 Teilen Toluol und 1 Teil Petrolether in reiner Form, mit einem Schmelzpunkt von 101-104°C, ausfällt.

Dieser Farbbildner ist im Kapselöl sehr gut löslich, zeigt keinerlei Sublimationstendenzen und entwickelt auf Zinksalicylat sofort eine rote Farbe.

Beispiel 5: 15,6 g 2′-Carboxy-2-hydroxy-4-diethylaminobenzophenon werden in 110 g Schwefelsäure 96 % bei 40°C gelöst. Bei 5-10°C trägt man innerhalb von 45 Minuten 12,6 g N-(3-Methoxyphenyl)-phthalimid ein, lässt in 30 Minuten die Temperatur auf 20°C steigen und giesst auf 500 ml Eiswasser. Die Suspension wird noch während 1 Stunde bei 20°C gerührt und dann abfiltriert, worauf die erhaltene Phthalidverbindung (28,8 g) gewaschen und getrocknet wird.

Hierauf wird die Phthalidverbindung zum Fluoranringschluss in 100 ml Xylol Isomerengemisch und 13,8 ml Triethylamin angeschlämmt und auf Rückfluss (115°C) geheizt. Man hält die Mischung 4 Stunden, wobei die Rückflusstemperatur auf 105°C sinkt. Man kühlt auf 20°C ab, gibt Wasser zu, trennt die Xylolphase ab und engt diese zur Trockene ein. Man erhält 25,8 g einer Verbindung der Formel 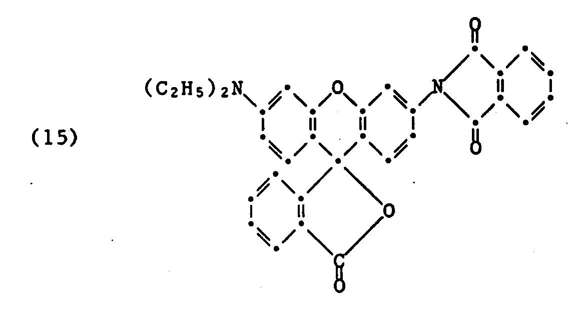
welche nach Umkristallisation aus Toluol einen Schmelzpunkt von 218-220°C aufweist. Dieser Farbbildner ist sublimierecht und entwickelt auf Phenolharz-CF sofort eine rote Farbe mit sehr guter Lichtechtheit.

Beispiel 6: Herstellung eines druckempfindlichen Kopierpapiers
Eine Lösung von 1 g der Fluoranverbindung der Formel (13) (Beispiel 3) in 80 g Diisopropylnaphthalin und 19 g Kerosin wird auf an sich bekannte Weise mit Gelatine und Gummi arabicum durch Koazervation mikroverkapselt, mit Stärkelösung vermischt und auf ein Blatt Papier gestrichen. Ein zweites Blatt Papier wird auf der Frontseite mit Aktivton als Farbentwickler beschichtet. Das erste, den sublimierechten Farbbildner enthaltende Blatt und das mit Farbentwickler beschichtete Papier werden mit den Beschichtungen benachbart aufeinandergelegt. Durch Schreiben mit der Hand oder mit der Schreibmaschine auf dem ersten Blatt wird Druck ausgeübt, und es entwickelt sich sofort auf dem mit dem Entwickler beschichteten Blatt eine intensiv rote Kopie, die ausgezeichnet lichtecht ist.

Beispiel 7: Ersetzt man in Beispiel 6 die Fluoranverbindung der Formel (13) durch eine Mischung aus
1 g der Fluoranverbindung der Formel (12),
0,8 g 3,3-Bis-(4′-dimethylaminophenyl)-6-dimethylaminophthalid
0,8 g N-Butylcarbazol-3-yl-bis-(4′-N-methyl-N-phenylaminophenyl)-methan und
2,4 g 6-Diethylamino-2-dibenzylaminofluoran
und verfährt im übrigen wie im Beispiel 6 beschrieben, so erhält man ein druckempfindliches Aufzeichnungsmaterial, welches durch Schreiben mit der Hand oder mit der Schreibmaschine eine intensive und lichtechte, schwarze Kopie ergibt.

Beispiel 8: 1 g der Fluoranverbindung der Formel (11) gemäss Beispiel 1 wird in 17 g Toluol gelöst. Zu dieser Lösung gibt man unter Rühren 12 g Polyvinylacetat, 8 g Calciumcarbonat und 2 g Titandioxid. Die erhaltene Suspension wird im Gewichtsverhältnis 1:1 mit Toluol verdünnt und mit einem 10 »m Rakel auf ein Blatt Papier gestrichen. Auf dieses Blatt Papier wird ein zweites Blatt Papier gelegt, dessen Unterseite bei einem Auftragsgewicht von 3 g/m² mit einer Mischung bestehend aus 1 Teil eines Amidwachses, 1 Teil eines Stearinwachses und 1 Teil Zinkchlorid beschichtet ist. Durch Schreiben mit der Hand oder mit der Schreibmaschine auf dem oberen Blatt wird Druck ausgeübt, und es entwickelt sich auf dem mit dem Farbbildner beschichteten Blatt sofort eine intensiv rotorange Farbe.

Beispiel 9: Herstellung eines wärmeempfindlichen Aufzeichnungsmaterials In einer Kugelmühle werden 32 g 4,4′-Isopropylidendiphenol (Bisphenol A), 3,8 g Distearylamid des Ethylendiamins, 39 g Kaolin, 20 g eines zu 88 % hydrolysierten Polyvinylalkohols und 500 ml Wasser gemahlen bis die Teilchengrösse ca. 5 »m beträgt. In einer zweiten Kugelmühle werden 6 g der Fluoranverbindung der Formel (12) gemäss Beispiel 2, 3 g eines zu 88 % hydrolysierten Polyvinylalkohols und 60 ml Wasser zu einer Teilchengrösse von ca. 3 »m gemahlen.

Die beiden Dispersionen werden zusammengegeben und mit einem Trockenauftragsgewicht von 5,5 g/m² auf ein Papier gestrichen. Durch Berührung des Papiers mit einem erhitzten Metallstift wird eine intensive, lichtechte, orange Farbe erhalten.

## Patentansprüche

1. 2- oder 3-Dicarbonsäureimid-Fluoranverbindungen der Formel 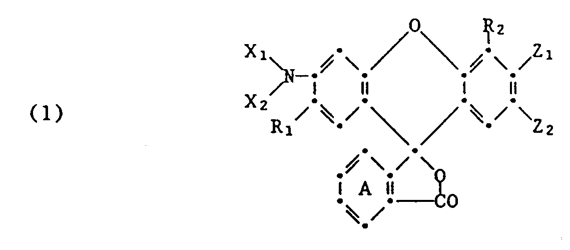 worin
R₁ und R₂, unabhängig voneinander, je Wasserstoff, Halogen, C₁-C₆-Alkyl oder C₁-C₆-Alkoxy,
von Z₁ und Z₂ eines die Dicarbonsäureimidgruppe 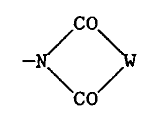 und das andere Wasserstoff, Halogen, C₁-C₆-Alkyl oder C₁-C₆-Alkoxy,
W einen gegebenenfalls durch Halogen substituierten, geradkettigen oder verzweigten Kohlenwasserstoffrest einer gesättigten oder ethylenisch ungesättigten Dicarbonsäure mit 4 bis 10 Kohlenstoffatomen, einen zweiwertigen cycloaliphatischen Rest oder den zweiwertigen Rest einer aromatischen Dicarbonsäure,
X₁ und X₂, unabhängig voneinander, je Wasserstoff, unsubstituiertes oder durch Halogen, Hydroxy, Cyano, Tetrahydrofuryl oder C₁-C₆-Alkoxy substituiertes Alkyl mit höchstens 12 Kohlenstoffatomen, Cycloalkyl mit 5 bis 10 Kohlenstoffatomen oder unsubstituiertes oder durch Halogen, Cyano, Nitro, C₁-C₆-Alkyl, C₁-C₆-Alkoxy, C₁-C₆-Alkoxycarbonyl, -NX'X'' oder 4-NX'X''-phenylamino substituiertes Benzyl oder Phenyl, worin X' und X'', unabhängig voneinander, Wasserstoff, C₁-C₆-Alkyl, Cyclohexyl, Benzyl oder Phenyl darstellen, oder
X₁ und X₂ zusammen mit dem sie verbindenden Stickstoffatom einen fünf- oder sechsgliedrigen, heterocyclischen Rest bedeuten und worin der Ring A unsubstituiert oder durch Halogen, Nitro, C₁-C₆-Alkyl, C₁-C₆-Alkoxy, C₁-C₆-Alkylthio, C₁-C₆-Alkoxycarbonyl, Amino, Mono-C₁-C₆-Alkylamino oder Di-C₁-C₆-Alkylamino substituiert ist.

2. Fluoranverbindungen gemäss Anspruch 1, dadurch gekennzeichnet, dass in Formel (1) R₁, R₂ und ein Z, unabhängig voneinander, je Wasserstoff, Methyl, Methoxy oder Chlor bedeuten.

3. Fluoranverbindungen gemäss einem der Ansprüche 1 und 2, dadurch gekennzeichnet, dass in Formel (1) X₁ und X₂, unabhängig voneinander, C₁-C₆-Alkyl, Cyclohexyl, Tolyl, Benzyl oder CyanoC₁-C₆-Alkyl oder -NX₁X₂ Pyrrolidinyl, N-C₁-C₆-Alkyl-tetrahydrofurfurylamino, 4-DiC₁-C₆-Alkylaminophenylamino oder 4-(4'-Phenylaminophenylamino)-phenylamino bedeuten.

4. Fluoranverbindungen gemäss einem der Ansprüche 1 bis 3, dadurch gekennzeichnet, dass in Formel (1) W einen gegebenenfalls durch Halogen substituierten, geradkettigen oder verzweigten Kohlenwasserstoffrest einer gesättigten oder ethylenisch-ungesättigten Dicarbonsäure mit 4 bis 10 Kohlenstoffatomen bedeutet.

5. Fluoranverbindungen gemäss einem der Ansprüche 1 bis 3, dadurch gekennzeichnet, dass in Formel (1) W den zweiwertigen Rest einer Tetrahydrophthalsäure, Hexahydrophthalsäure, Phthalsäure oder Naphthalindicarbonsäure bedeutet.

6. Fluoranverbindungen gemäss einem der Ansprüche 1 bis 3, dadurch gekennzeichnet, dass in Formel (1) W einen Phenylenrest darstellt, der unsubstituiert oder durch Halogen, Nitro, C₁-C₆-Alkyl, C₁-C₆-Alkoxy oder C₁-C₆-Alkoxycarbonyl substituiert ist.

7. Fluoranverbindungen gemäss einem der Ansprüche 1 bis 6, dadurch gekennzeichnet, dass in Formel (1) der Ring A unsubstituiert oder durch Halogen, Nitro, C₁-C₆-Alkyl, C₁-C₆-Alkoxy oder DiC₁-C₆-Alkylamino substituiert ist.

8. Fluoranverbindungen gemäss Anspruch 1, dadurch gekennzeichnet, dass sie der Formel 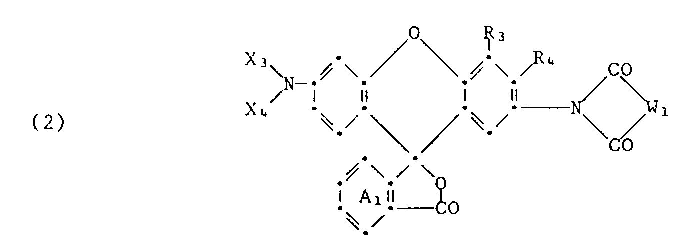 entsprechen, worin
W₁ Alkylen oder Alkenylen mit 2 bis 4 Kohlenstoffatomen, Cyclohexylen oder Phenylen, das unsubstituiert oder durch Halogen, Methyl, Methoxy oder Carbomethoxy substituiert ist,
R₃ und R₄, unabhängig voneinander, je Wasserstoff, Halogen oder C₁-C₆-Alkyl,
X₃ C₁-C₆-Alkyl, C₅-C₆-Cycloalkyl, Benzyl, Phenyl oder durch Halogen, C₁-C₄-Alkyl oder C₁-C₄-Alkoxy substituiertes Phenyl,
X₄ C₁-C₆-Alkyl oder Benzyl oder
-NX₃X₄ Pyrrolidinyl, Piperidinyl, Morpholinyl oder
N-C₁-C₆-Alkyl-N-tetrahydrofurfurylamino bedeuten und worin
der Benzolring A₁ unsubstituiert oder durch Halogen, C₁-C₆-Alkyl oder C₁-C₆-Alkoxy substituiert ist.

9. Fluoranverbindungen gemäss Anspruch 8, dadurch gekennzeichnet, dass in Formel (2)
W₁ unsubstituiertes oder durch Chlor substituiertes Phenylen,
X₃ und X₄ C₁-C₄-Alkyl,
R₃ und R₄ Wasserstoff, Methyl oder Chlor bedeuten und der Ring A₁ unsubstituiert ist.

10. Fluoranverbindungen gemäss Anspruch 1, dadurch gekennzeichnet, dass sie der Formel 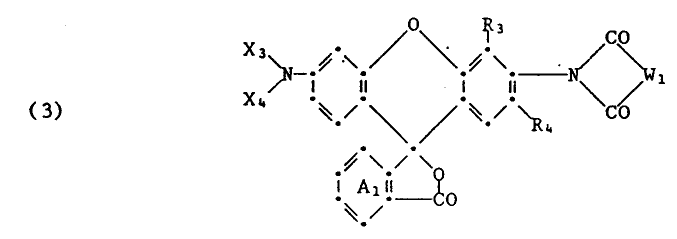 entsprechen, worin
W₁ Alkylen oder Alkenylen mit 2 bis 4 Kohlenstoffatomen, Cyclohexylen oder Phenylen, das unsubstituiert oder durch Halogen, Methyl, Methoxy oder Carbomethoxy substituiert ist,
R₃ und R₄, unabhängig voneinander, je Wasserstoff, Halogen oder C₁-C₆-Alkyl,
X₃ C₁-C₆-Alkyl, C₅-C₆-Cycloalkyl, Benzyl, Phenyl oder durch Halogen, C₁-C₄-Alkyl oder C₁-C₄-Alkoxy substituiertes Phenyl,
X₄ C₁-C₆-Alkyl oder Benzyl oder
-NX₃X₄ Pyrrolidinyl, Piperidinyl, Morpholinyl oder
N-C₁-C₆-Alkyl-N-tetrahydrofurfurylamino bedeuten und worin
der Benzolring A₁ unsubstituiert oder durch Halogen, C₁-C₆-Alkyl oder C₁-C₆-Alkoxy substituiert ist.

11. Fluoranverbindungen gemäss Anspruch 10, dadurch gekennzeichnet, dass in Formel (3)
W₁ unsubstituiertes oder durch Chlor substituiertes Phenylen,
X₃ und X₄ C₁-C₄-Alkyl,
R₃ und R₄ Wasserstoff, Methyl oder Chlor bedeuten und der Ring A₁ unsubstituiert ist.

12. Fluoranverbindungen gemäss Anspruch 1, dadurch gekennzeichnet, dass sie der Formel 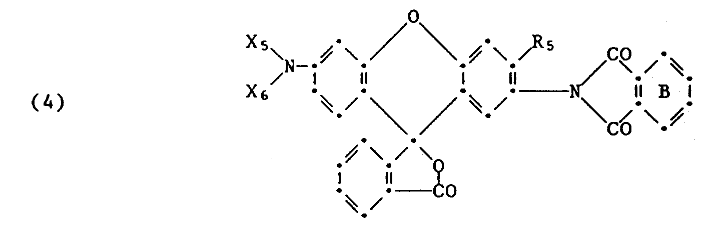 entsprechen,
worin
R₅ Wasserstoff oder Methyl,
X₅ C₁-C₄-Alkyl, Cyclohexyl oder Tolyl
und
X₆ C₁-C₄-Alkyl bedeuten und der Ring B unsubstituiert oder durch 1 bis 4 Chloratome substituiert ist.

13. Verfahren zur Herstellung von Dicarbonsäureimid-Fluoranverbindungen gemäss Anspruch 1, dadurch gekennzeichnet, dass man eine Ketosäureverbindung der Formel 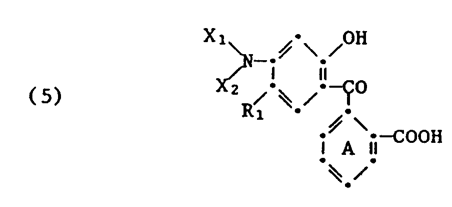 worin A, X₁, X₂ und R₁ die in Anspruch 1 angegebene Bedeutung haben, mit einer die Imidgruppe enthaltenden Verbindung der Formel 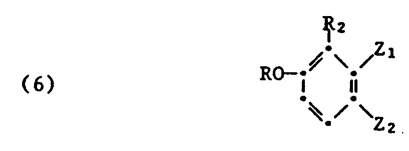 worin R₂, Z₁ und Z₂ die in Anspruch 1 angegebene Bedeutung haben und R Wasserstoff oder Methyl bedeutet, umsetzt.

14. Verfahren zur Herstellung von Dicarbonsäureimid-Fluoranverbindungen gemäss Anspruch 1, dadurch gekennzeichnet, dass man eine Amino-Fluoranverbindung der Formel 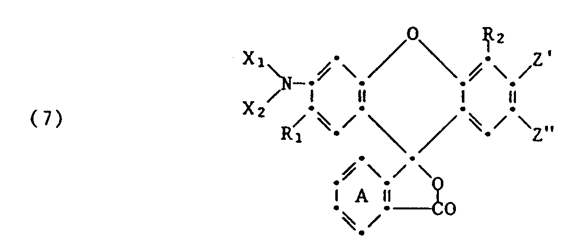 worin von Z' und Z'' eines -NH₂ und das andere Wasserstoff, Halogen, C₁-C₆-Alkyl oder C₁-C₆-Alkoxy bedeuten und R₁, R₂, X₁, X₂ und A die in Anspruch 1 angegebene Bedeutung haben, mit einem Dicarbonsäureanhydrid der Formel 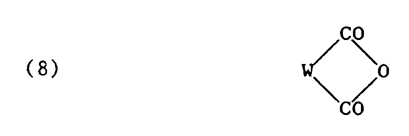 worin W die in Anspruch 1 angegebene Bedeutung hat, umsetzt.

15. Verwendung von Dicarbonsäureimid-Fluoranverbindungen gemäss einem der Ansprüche 1 bis 12 als Farbbildner in einem druckempfindlichen oder wärmeempfindiichen Aufzeichnungsmaterial.

16. Druck- oder wärmeempfindliches Aufzeichnungsmaterial, dadurch gekennzeichnet, dass es in seinem Farbreaktantensystem als Farbbildner mindestens eine Fluoranverbindung gemäss einem der Ansprüche 1 bis 12 enthält.

17. Druckempfindliches Aufzeichnungsmaterial gemäss Anspruch 16, dadurch gekennzeichnet, dass es die Fluoranverbindung, gelöst in einem organischen Lösungsmittel, und mindestens einen festen Elektronenakzeptor enthält.

18. Druckempfindliches Aufzeichnungsmaterial gemäss einem der Ansprüche 16 und 17, dadurch gekennzeichnet, dass die Fluoranverbindung in Mikrokapseln eingekapselt ist.

19. Druckempfindliches Aufzeichnungsmaterial gemäss Anspruch 18, dadurch gekennzeichnet, dass die eingekapselte Fluoranverbindung in Form einer Schicht auf der Rückseite eines Uebertragungsblattes und der Elektronenakzeptor in Form einer Schicht auf der Vorderseite des Empfangsblattes vorhanden sind.

20. Wärmeempfindliches Aufzeichnungsmaterial gemäss Anspruch 16, dadurch gekennzeichnet, dass es in mindestens einer Schicht mindestens eine Fluoranverbindung gemäss einem der Ansprüche 1 bis 12, einen Elektronenakzeptor und gegebenenfalls ein Bindemittel und/oder Wachs enthält.

21. Aufzeichnungsmaterial gemäss einem der Ansprüche 16 bis 20, dadurch gekennzeichnet, dass die Fluoranverbindung gemeinsam mit einem oder mehreren anderen Farbbildnern enthalten ist.

## Claims

1. A 2- or 3-dicarboximidofluoran compound of the formula 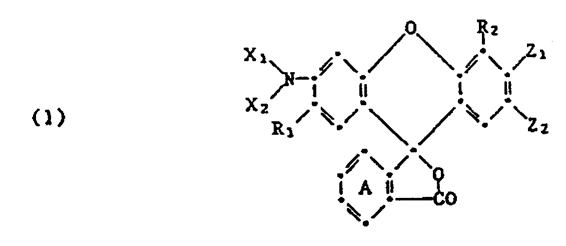 in which R₁ and R₂ independently of one another are each hydrogen, halogen, C₁-C₆alkyl or C₁-C₆alkoxy, one of Z₁ and Z₂ is the dicarboximido group 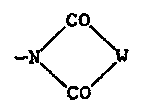 and the other is hydrogen, halogen, C₁-C₆alkyl or C₁-C₆alkoxy, W is an unsubstituted or halogen-substituted straight-chain or branched hydrocarbon radical of a saturated or ethylenically unsaturated dicarboxylic acid having 4 to 10 carbon atoms, a divalent cycloaliphatic radical or the divalent radical of an aromatic dicarboxylic acid, X₁ and X₂ independently of one another are each hydrogen, alkyl which has not more than 12 carbon atoms and is unsubstituted or substituted by halogen, hydroxyl, cyano, tetrahydrofuryl or C₁-C₆alkoxy, cycloalkyl which has 5 to 10 carbon atoms, or benzyl or phenyl each of which is unsubstituted or substituted by halogen, cyano, nitro, C₁-C₆alkyl, C₁-C₆alkoxy, C₁-C₆alkoxycarbonyl, -NX'X'' or -4-NX'X''-phenylamino in which X' and X'' independently of one another are hydrogen, C₁-C₆alkyl, cyclohexyl, benzyl or phenyl, or X₁ and X₂, together with the nitrogen atom linking them, are a five-membered or six-membered heterocyclic radical, and in which the ring A is unsubstituted or substituted by halogen, nitro, C₁-C₆alkyl, C₁-C₆alkoxy, C₁-C₆alkylthio, C₁-C₆alkoxycarbonyl, amino, mono-C₁-C₆alkylamino or di-C₁-C₆alkylamino.

2. A fluoran compound according to claim 1, wherein R₁, R₂ and one Z in formula (1) independently of one another are each hydrogen, methyl, methoxy or chlorine.

3. A fluoran compound according to one of claims 1 and 2, wherein X₁ and X₂ in formula (1) independently of one another are C₁-C₆alkyl, cyclohexyl, tolyl, benzyl or cyano-C₁-C₆alkyl, or -NX₁X₂ is pyrrolidinyl, N-C₁-C₆alkyl-tetrahydrofurfurylamino, 4-di-C₁-C₆alkyl-aminophenylamino or 4-(4'-phenylaminophenylamino)phenylamino.

4. A fluoran compound according to one of claims 1 to 3, wherein W in formula (1) is an unsubstituted or halogen-substituted straight-chain or branched hydrocarbon radical of a saturated or ethylenically unsaturated dicarboxylic acid having 4 to 10 carbon atoms.

5. A fluoran compound according to one of claims 1 to 3, wherein W in formula (1) is the divalent radical of a tetrahydrophthalic acid, hexahydrophthalic acid, phthalic acid or naphthalenedicarboxylic acid.

6. A fluoran compound according to one of claims 1 to 3, wherein W in formula (1) is a phenylene radical which is unsubstituted or substituted by halogen, nitro, C₁-C₆alkyl, C₁-C₆alkoxy or C₁-C₆alkoxycarbonyl.

7. A fluoran compound according to one of claims 1 to 6, wherein the ring A in formula (1) is unsubstituted or substituted by halogen, nitro, C₁-C₆alkyl, C₁-C₆alkoxy or di-C₁-C₆alkylamino.

8. A fluoran compound according to claim 1, which has the formula 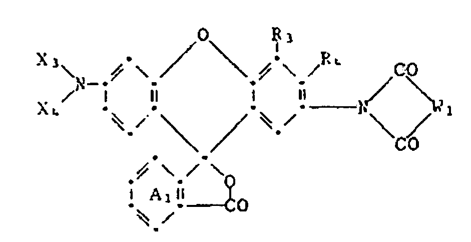 in which W₁ is alkylene or alkenylene having 2 to 4 carbon atoms, cyclohexylene or phenylene which is unsubstituted or is substituted by halogen, methyl, methoxy or carbomethoxy, R₃ and R₄ independently of one another are each hydrogen, halogen or C₁-C₆alkyl, X₃ is C₁-C₆alkyl, C₅-C₆cycloalkyl, benzyl, phenyl or phenyl which is substituted by halogen, C₁-C₄alkyl or C₁-C₄alkoxy, X₄ is C₁-C₆alkyl or benzyl or -NX₃X₄ is pyrrolidinyl, piperidinyl, morpholinyl or N-C₁-C₆alkyl-N-tetrahydrofurfurylamino and in which the benzene ring A₁ is unsubstituted or is substituted by halogen, C₁-C₆alkyl or C₁-C₆alkoxy.

9. A fluoran compound according to claim 8, wherein in formula (2) W₁ is phenylene which is unsubstituted or is substituted by chlorine, X₃ and X₄ are C₁-C₄alkyl, R₃ and R₄ are hydrogen, methyl or chlorine and the ring A₁ is unsubstituted.

10. A fluoran compound according to claim 1, which has the formula 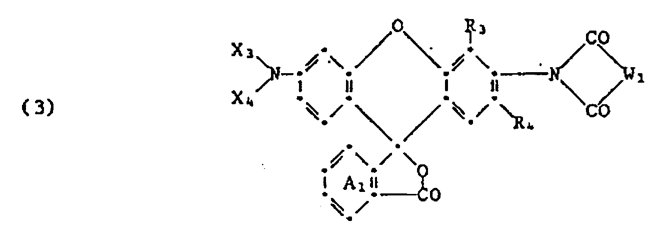 in which W₁ is alkylene or alkenylene having 2 to 4 carbon atoms, cyclohexylene or phenylene which is unsubstituted or is substituted by halogen, methyl, methoxy or carbomethoxy, R₃ and R₄ independently of one another are each hydrogen, halogen or C₁-C₆alkyl, X₃ is C₁-C₆alkyl, C₅-C₆cycloalkyl, benzyl, phenyl or phenyl which is substituted by halogen, C₁-C₄alkyl or C₁-C₄alkoxy, X₄ is C₁-C₆alkyl or benzyl or -NX₃X₄ is pyrrolidinyl, piperidinyl, morpholinyl or N-C₁-C₆alkyl-N-tetrahydrofurfurylamino and in which the benzene ring A₁ is unsubstituted or is substituted by halogen, C₁-C₆alkyl or C₁-C₆alkoxy.

11. A fluoran compound according to claim 10, wherein in formula (3) W₁ is phenylene which is unsubstituted or is substituted by chlorine, X₃ and X₄ are C₁-C₄alkyl, R₃ and R₄ are hydrogen, methyl or chlorine and the ring A₁ is unsubstituted.

12. A fluoran compound according to claim 1, which has the formula 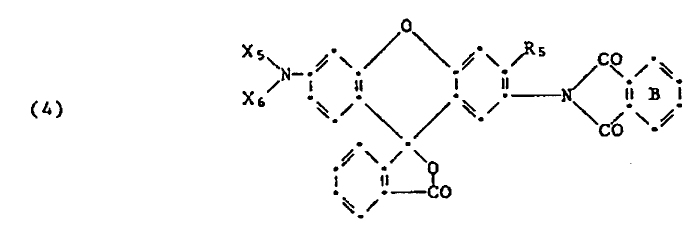 in which R₅ is hydrogen or methyl, X₅ is C₁-C₄alkyl, cyclohexyl or tolyl and X₆ is C₁-C₄alkyl and the ring B is unsubstituted or substituted by 1 to 4 chlorine atoms.

13. A process for the preparation of a dicarboximidofluoran compound according to claim 1, which comprises reacting a keto acid compound of the formula 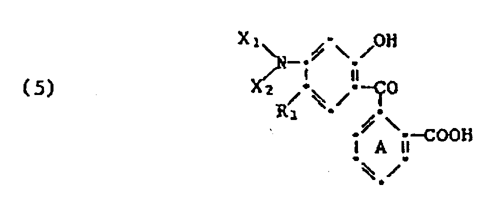 in which A, X₁, X₂ and R₁ are as defined in claim 1,
with a compound, containing the imide group, of the formula 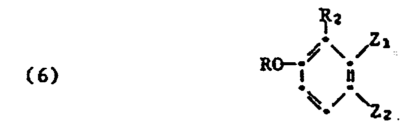 in which R₂, Z₁ and Z₂ are as defined in claim 1 and R is hydrogen or methyl.

14. A process for the preparation of a dicarboximidofluoran compound according to claim 1, which comprises reacting an aminofluoran compound of the formula 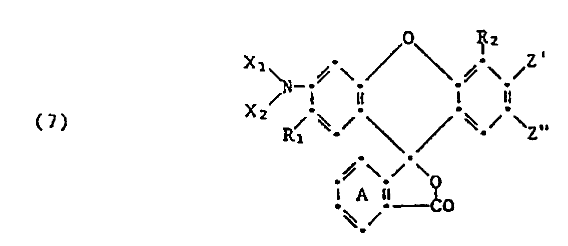 in which one of Z' and Z'' is -NH₂ and the other is hydrogen, halogen, C₁-C₆alkyl or C₁-C₆alkoxy and R₁, R₂, X₁, X₂ and A are as defined in claim 1, with a dicarboxylic anhydride of the formula 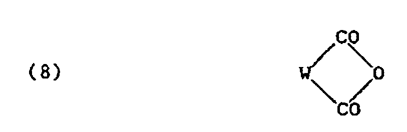 in which W is as defined in claim 1.

15. The use of a dicarboximidofluoran compound according to one of claims 1 to 12 as a colour former in a pressure-sensitive or heat-sensitive recording material.

16. A pressure-sensitive or heat-sensitive recording material, which contains at least one fluoran compound according to one of claims 1 to 12 as colour former in its colour reactant system.

17. A pressure-sensitive recording material according to claim 16, which contains the fluoran compound, dissolved in an organic solvent, and at least one solid electron acceptor.

18. A pressure-sensitive recording material according to one of claims 16 and 17, wherein the fluoran compound is encapsulated in microcapsules.

19. A pressure-sensitive recording material according to claim 18, wherein the encapsulated fluoran compound is present in the form of a layer on the reverse side of a transfer sheet and the electron acceptor is present in the form of a layer on the front side of the receiver sheet.

20. A heat-sensitive recording material according to claim 16, which contains, in at least one layer, at least one fluoran compound according to one of claims 1 to 12, an electron acceptor and, if desired, a binder and/or wax.

21. A recording material according to one of claims 16 to 20, wherein the fluoran compound is present together with one or more other colour formers.

## Revendications

1. Composés de type 2- ou 3-dicarboximidofluoranne, de formule : 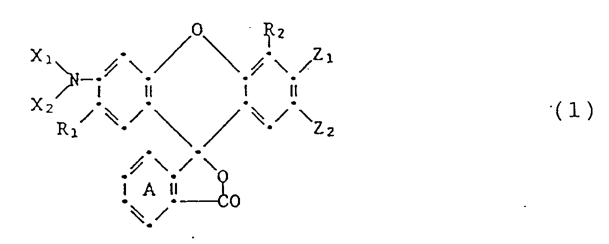 dans laquelle
R₁ et R₂ représentent chacun, indépendamment l'un de l'autre, un atome d'hydrogène ou d'halogène ou un groupe alkyle en C₁-C₆ ou alcoxy en C₁-C₆,
l'un des symboles Z₁ et Z₂ représente un groupe dicarboximide 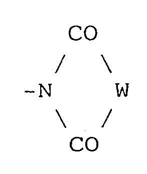 et l'autre représente un atome d'hydrogène ou d'halogène ou un groupe alkyle en C₁-C₆ ou alcoxy en C₁-C₆,
W représente le résidu hydrocarboné, à chaîne droite ou ramifiée, portant éventuellement un atome d'halogène en tant que substituant, d'un acide dicarboxylique saturé ou à insaturation éthylénique, comportant de 4 à 10 atomes de carbone, un résidu cycloaliphatique divalent ou le résidu divalent d'un acide dicarboxylique aromatique,
X₁ et X₂ représentent chacun, indépendamment l'un de l'autre, un atome d'hydrogène, un groupe alkyle comportant au plus 12 atomes de carbone et portant ou non des substituants halogéno, hydroxy, cyano, tétrahydrofuryle ou alcoxy en C₁-C₆, un groupe cycloalkyle comportant de 5 à 10 atomes de carbone ou un groupe benzyle ou phényle portant ou non des substituants halogéno, cyano, nitro, alkyle en C₁-C₆, alcoxy en C₁-C₆, (alcoxy en C₁-C₆)carbonyle, -NX'X'' ou 4-NX'X''-phénylamino, où X' et X'' représentent chacun, indépendamment l'un de l'autre, un atome d'hydrogène ou un groupe alkyle en C₁-C₆, cyclohexyle, benzyle ou phényle, ou bien
X₁ et X₂ représentent, conjointement avec l'atome d'azote qui les relie, un résidu hétérocyclique à cinq ou six chaînons,
et le cycle A porte ou non des substituants halogéno, nitro, alkyle en C₁-C₆, alcoxy en C₁-C₆, alkylthio en C₁-C₆, (alcoxy en C₁-C₆)carbonyle, amino, mono(alkyl en C₁-C₆)-amino ou di(alkyl en C₁-C₆)amino.

2. Composés de type fluoranne, conformes à la revendication 1, caractérisés en ce que, dans la formule (1), R₁, R₂ et l'un des symboles Z, indépendamment l'un de l'autre, représentent chacun un atome d'hydrogène ou de chlore ou un groupe méthyle ou méthoxy.

3. Composés de type fluoranne, conformes à l'une des revendications 1 et 2, caractérisés en ce que, dans la formule (1), X₁ et X₂ représentent chacun, indépendamment l'un de l'autre, un groupe alkyle en C₁-C₆, cyclohexyle, tolyle, benzyle ou cyanoalkyle en C₁-C₆, ou bien le groupe représenté par -NX₁X₂ est un groupe pyrrolidinyle, N-(alkyl en C₁-C₆)-tétrahydrofurfurylamino, 4-di(alkyl en C₁-C₆)amino-phénylamino ou 4-(4'-phénylamino-phénylamino)phénylamino.

4. Composés de type fluoranne, conformes à l'une des revendications 1 à 3, caractérisés en ce que, dans la formule (1), W représente le reste hydrocarboné à chaîne droite ou ramifiée, portant éventuellement des atomes d'halogène en tant que substituants, d'un acide dicarboxylique saturé ou à insaturation éthylénique, comportant de 4 à 10 atomes de carbone.

5. Composés de type fluoranne, conformes à l'une des revendications 1 à 3, caractérisés en ce que, dans la formule (1), W représente le reste divalent d'un acide tétrahydrophtalique, hexahydrophtalique, phtalique ou naphtalène-dicarboxylique.

6. Composés de type fluoranne, conformes à l'une des revendications 1 à 3, caractérisés en ce que, dans la formule (1), W représente un groupe phénylène portant ou non des substituants halogéno, nitro, alkyle en C₁-C₆, alcoxy en C₁-C₆ ou (alcoxy en C₁-C₆)-carbonyle.

7. Composés de type fluoranne, conformes à l'une des revendications 1 à 6, caractérisés en ce que, dans la formule (1), le cycle A porte ou non des substituants halogéno, nitro, alkyle en C₁-C₆, alcoxy en C₁-C₆ ou di(alkyl en C₁-C₆)-amino.

8. Composés de type fluoranne, conformes à la revendication 1, caractérisés en ce qu'ils correspondent à la formule : 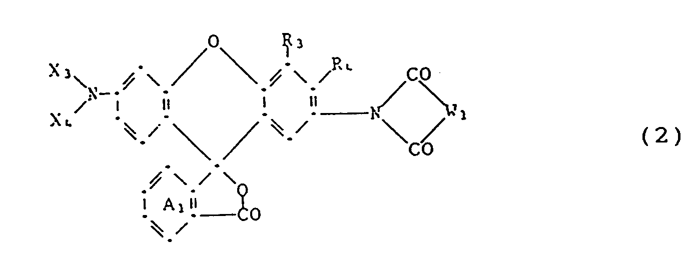 dans laquelle
W₁ représente un groupe alkylène ou alcénylène comportant de 2 à 4 atomes de carbone, cyclohexylène ou phénylène, qui porte ou non des substituants halogéno, méthyle, méthoxy ou carbométhoxy,
R₃ et R₄ représentent chacun, indépendamment l'un de l'autre, un atome d'hydrogène ou d'halogène ou un groupe alkyle en C₁-C₆,
X₃ représente un groupe alkyle en C₁-C₆, cycloalkyle en C₅-C₆, benzyle, phényle ou phényle portant des substituants halogéno, alkyle en C₁-C₄ ou alcoxy en C₁-C₄,
X₄ représente un groupe alkyle en C₁-C₆ ou benzyle,
ou bien -NX₃X₄ représente un groupe pyrrolidinyle, pipéridinyle, morpholinyle ou encore N-(alkyl en C₁-C₆)-N-tétrahydrofurfuryl-amino, et
le cycle benzénique A₁ ne porte pas de substituant ou porte, en tant que substituants, des atomes d'halogène ou des groupes alkyle en C₁-C₆ ou alcoxy en C₁-C₆.

9. Composés de type fluoranne, conformes à la revendication 8, caractérisés en ce que, dans la formule (2), W₁ représente un groupe phénylène portant ou non des substituants chloro, X₃ et X₄ représentent des groupes alkyle en C₁-C₄, R₃ et R₄ représentent chacun un atome d'hydrogène ou de chlore ou un groupe méthyle, et le cycle A₁ ne porte pas de substituant.

10. Composés de type fluoranne, conformes à la revendication 1, caractérisés en ce qu'ils correspondent à la formule : 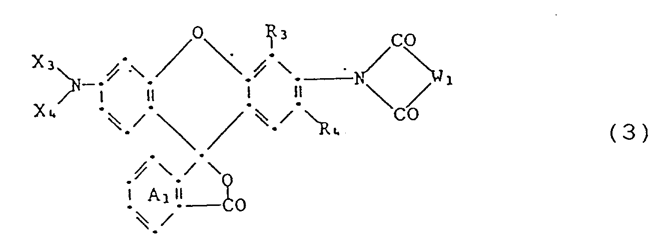 dans laquelle
W₁ représente un groupe alkylène ou alcénylène comportant de 2 à 4 atomes de carbone, cyclohexylène ou phénylène, qui porte ou non des substituants halogéno, méthyle, méthoxy ou carbuméthoxy,
R₃ et R₄ représentent chacun, indépendamment l'un de l'autre, un atome d'hydrogène ou d'halogène ou un groupe alkyle en C₁-C₆,
X₃ représente un groupe alkyle en C₁-C₆, cycloalkyle en C₅-C₆, benzyle, phényle ou phényle portant des substituants halogéno, alkyle en C₁-C₄ ou alcoxy en C₁-C₄,
X₄ représente un groupe alkyle en C₁-C₆ ou benzyle,
ou bien -NX₃X₄ représente un groupe pyrrolidinyle, pipéridinyle, morpholinyle ou encore N-(alkyl en C₁-C₆)-N-tétrahydrofurfuryl-amino, et
le cycle benzénique A₁ ne porte pas de substituant ou porte, en tant que substituants, des atomes d'halogène ou des groupes alkyle en C₁-C₆ ou alcoxy en C₁-C₆.

11. Composés de type fluoranne, conformes à la revendication 10, caractérisés en ce que, dans la formule (3), W₁ représente un groupe phénylène portant ou non des substituants chloro, X₃ et X₄ représentent des groupes alkyle en C₁-C₄, R₃ et R₄ représentent chacun un atome d'hydrogène ou de chlore ou un groupe méthyle, et le cycle A₁ ne porte pas de substituant.

12. Composés de type fluoranne, conformes à la revendication 1, caractérisés en ce qu'ils correspondent à la formule : 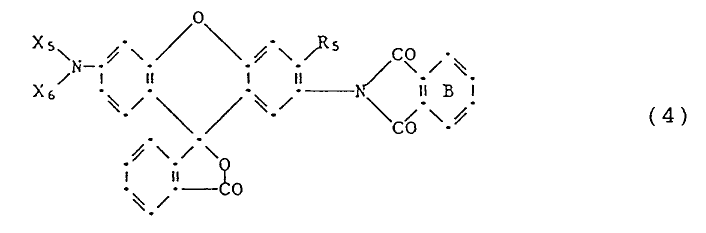 dans laquelle
R₅ représente un atome d'hydrogène ou un groupe méthyle,
X₅ représente un groupe alkyle en C₁-C₄, cyclohexyle ou tolyle,
X₆ représente un groupe alkyle en C₁-C₄,
et le cycle B ne porte pas de substituant ou porte de 1 à 4 atomes de chlore en tant que substituants.

13. Procédé de préparation de composés de type dicarboximidofluoranne, conformes à la revendication 1, caractérisé en ce que l'on fait réagir un cétoacide de formule : 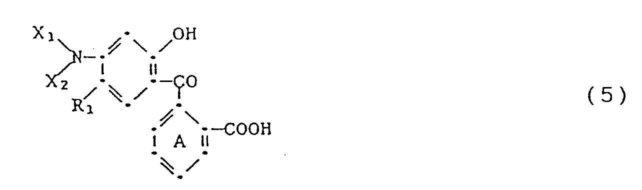 dans laquelle A, X₁, X₂ et R₁ ont les significations indiquées dans la revendication 1,
avec un composé contenant un groupe fonctionnel imide, de formule : 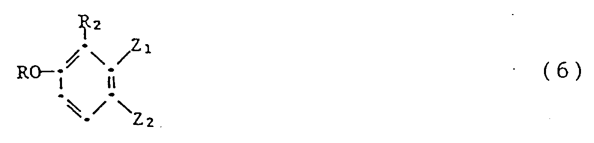 dans laquelle R₂, Z₁ et Z₂ ont les significations indiquées dans la revendication 1 et R représente un atome d'hydrogène ou un groupe méthyle.

14. Procédé de préparation de composés de type dicarboximidofluoranne, conformes à la revendication 1, caractérisé en ce que l'on fait réagir un composé de type aminofluoranne, de formule : 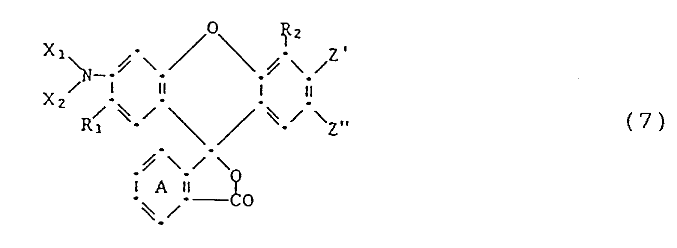 dans laquelle l'un des symboles Z' et Z'' représente un groupe amino et l'autre représente un atome d'hydrogène ou d'halogène ou un groupe alkyle en C₁-C₆ ou alcoxy en C₁-C₆, et R₁, R₂, X₁, X₂ et A ont les significations indiquées dans la revendication 1,
avec un anhydride d'acide dicarboxylique, de formule : 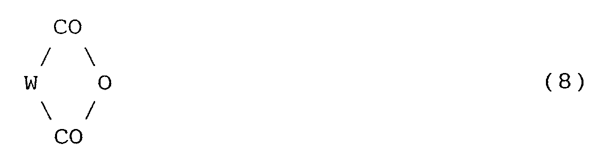 dans laquelle W a la signification indiquée dans la revendication 1.

15. Utilisation des composés de type dicarboximidofluoranne conformes à l'une des revendications 1 à 12, en tant qu'agents chromogènes dans un matériau d'enregistrement sensible à la pression ou à la chaleur.

16. Matériau d'enregistrement sensible à la pression ou la chaleur, caractérisé en ce qu'il contient dans son système de réactifs donnant une couleur, en tant qu'agent chromogène, au moins un dérivé de fluoranne conforme à l'une des revendications 1 à 12.

17. Matériau d'enregistrement sensible à la pression, conforme à la revendication 16, caractérisé en ce qu'il contient le dérivé de fluoranne, dissous dans un solvant organique, et au moins un accepteur d'électrons solide.

18. Matériau d'enregistrement sensible à la pression, conforme à l'une des revendications 16 et 17, caractérisé en ce que le dérivé de fluoranne est encapsulé dans des microcapsules.

19. Matériau d'enregistrement sensible à la pression, conforme à la revendication 18, caractérisé en ce que le dérivé de fluoranne encapsulé se trouve sous la forme d'une couche étalée au verso d'une feuille de transfert et l'accepteur d'électrons se trouve sous la forme d'une couche étalée au recto de la feuille réceptrice.

20. Matériau d'enregistrement sensible à la chaleur, conforme à la revendication 16, caractérisé en ce qu'il contient, dans au moins une couche, au moins un dérivé de fluoranne conforme à l'une des revendications 1 à 12, un accepteur d'électrons, et éventuellement un liant et/ou une cire.

21. Matériau d'enregistrement conforme à l'une des revendications 16 à 20, caractérisé en ce qu'il contient, conjointement avec le dérivé de fluoranne, un ou plusieurs autres agents chromogènes.
